# EUROPEAN PATENT APPLICATION

(11) **EP 3 892 243 A1**
(43) Date of publication of application: **13.10.2021**
(21) Application number: 20382279.6
(22) Date of filing: 07.04.2020
(51) Int. Cl.: A61F 5/56

(54) **DEVICE FOR THE SNORING AND SLEEPING APNEA AND HYPOAPNEA**

(71) Applicant: Garcia Gonzalez, Luis Amando, 33529 Nava (Asturias) (ES)
(72) Inventor: Garcia Gonzalez, Luis Amando, 33529 Nava (Asturias) (ES)
(74) Representative: Pons

(57) **Abstract**

Device for snoring, sleep apnoea and hypoapnoea that achieves opening of a user's upper airway, reconstructing the loss of the muscular tone of the palate and tongue, and which comprises a supporting splint (1), and at least one of: a front attraction group, comprising a front magnet (4) fixed to the support splint (1), and a lingual implant (9), intended to be implanted in the tongue of a user, existing between the front magnet (4) and the lingual implant (9) an attraction force; a posterior attraction group, comprising one or more affixed posterior magnets (7) to the supporting splint (1), and a palatal implant (8), intended to be implanted in the palate of the user, there being an attractive force between the posterior magnets (7) and the palatal implant (8).

## Description

### OBJECT OF THE INVENTION

The object of the invention is a device for the reduction of snoring and the sleep apnoea and hipoapnoea, that reaches the opening of the superior air tract of a user, restoring the lose muscle tone which is a triggered factor for the superior air tract collapse because of the tissue pressure lessen.

### BACKGROUND OF THE INVENTION

Nowadays it is estimated that the 4-6% of the adult population has the Obstructive Sleep Apnoea Syndrome (OSAS). This prevalence increases until a 25% in the population older than 60 years.

Those last years the awareness about the importance of the respiratory (or breathe) sleep disorders in relation with health, quality of life and economic costs even direct or indirect has been increasing. What's more, the indirect consequences of OSAS are getting revealed in an increasing of workplace and road accidents.

Many clinic studies have connected OSAS with arterial hypertension, ischemic heart disease, pulmonary hypertension, cerebrovascular accidents, insulin resistance syndrome, depression or sexual dysfunction. It is also related with rising of work absenteeism, temporary inability to work and ambulatory sanitary spend like the admissions of patients with this pathology.

It has been postulated a genetic factor in the appearance of OSAS, in addition, risk factors as sedentary lifestyle, obesity, the alcohol, tobacco, caffeine, benzodiazepines, antidepressants and muscle relaxants consume before sleep time are responsible for its growing incidence.

The inspiration collapse is given by the obstruction made for the negative intrathoracic pressure generated by the diaphragm and the respiratory musculature. The thoracic negative pressure produces the closure of soft tissues with less support and more distensible, located in the soft palate, the base of the tongue and the pharynx lateral walls or epiglottis, especially during the muscular hypotonia in the deepest sleep stages (REM and not REM stage).

Any therapeutic step with an OSAS patient has to go first of all with changes in the lifestyle avoiding the overweight and all kind of substances that disrupt the sleep quality.

Besides, is very common the use of CPAP (Continuous Positive Airway Pressure). Actually, this therapy offers, in the cases in which is tolerated, an unquestionable improvement, reducing the diary drowsiness, the microwaking ups, avoiding the sleep fragmentation with a better arterial tension control and decreasing the traffic accidents risk.

Nevertheless, the acceptation CPAP rate is about 56% of the users. A 32% do not tolerate or reject from the beginning the CPAP treatment, a 31% start its use but give up in the next 12 months. Many patients do not tolerate the discomfort from the air continuous pressure introduction of the mask that causes in many cases rhinitis and an increasing of nasal resistance, nasal dryness symptoms, pharynx, morning cephalous, the operation of the machine noise, etc.

On the other hand, we must consider that is a method which does not deal with the source of the obstruction; it is symptomatic and only works in the moments when it's used.

CPAP's usability has been consolidated because of the surgical morbidity techniques that had been used as an alternative to OSAS treatment.

Biggest surgical treatment problems are the tongue base obstructions. In those cases the surgery (orthognathic surgery, tongue base resection) has a high morbidity and high economic costs so it is a non-efficient option and with less patients acceptation.

Other use techniques are the advancement of the genioglossus muscle or the hyoid suspension. Both are surgical interventions with long hospital admissions, annoying pre-operative and the possibility of changes in the phonation and the swallowing characters.

The only technique that solves the OSAS is the tracheostomy but obviously its morbidity and social and personal impact made its use punctual.

Other alternatives are intraoral devices that are divided into palate raiser, linear tongue retainers and specially mandibular advancement device, despite they do not need surgical procedures they are not exempted from secondary effects as the pain and the dysfunction in the temporomandibular joint, the change in the bite and the loss of dentin. Its use is impossible in edentulous patients.

### DESCRIPTION OF THE INVENTION

The sleep apnoea and hipoapnoea device, object of the present invention, is guide to the anterior displacement of the structures that collapse the airway because of its fall towards the posterior pharyngeal wall, in users suffering from snoring, sleep apnoea or hypoapnea.

Specifically, it is a device that reaches the superior airway opened, anchoring the soft tissues without skeletal support in normal conditions to a structure with an adequate and enough support to avoid the collapse against negative inhale pressures. It involves the rebuild the muscle tone lose which is a triggered factor of the superior airway collapse because of the tissue pressure lessen.

For that, the sleep apnoea and hipoapnoe device comprises a support splint intended to be positioned between both hemi arches (dental hemiarches) of the user, completely personalized with its bite characters and its temporomandibular joint movement.

The device also comprises a front attraction group which in turn comprises a front magnet fixed on the support splint and a tongue intended to be implanted in the user's tongue, producing a force of attraction between the front magnet and the tongue implant.

Besides, the device can comprise a posterior attraction group that comprises one or more posterior magnets fixed on the support splint and a palatal implant intended to be implanted in the users' palate, creating an attraction force between the posterior magnets and the palate implant.

In a first aspect of the invention, the device just comprises the front attraction group if the user suffers a base tongue collapse. In a second aspect of the invention, the device just comprises the posterior attraction group, if the user suffers a palatine collapse. In a third aspect of the invention, when the user suffers a tongue, lateral walls and palate collapse the device comprises both the posterior attraction group and the front attraction group. The requirement to place one or two implants will depend on the collapse localization that causes the snoring.

In a fourth aspect of the invention, the front magnet or/and the posterior magnets could be electromagnets which turn on or turn off among which flow an electric current, so once the device is inside the users' mouth the electromagnets activation can be controlled; they will be in compliance with the safety regulation currently in force on the subject.

In the fourth aspect of the invention, the device comprises feeding means fixed on the support splint, connected with the front magnet and/or the posterior magnets and a controller fixed on the support splint and in relation with the feeding means, the front magnet and the posterior magnets.

In a fifth aspect of the invention, the device comprises a matrix reservoir fixed on the support splint, one or more posterior reservoirs assembled on the posterior magnets, positioned between those magnets and the palatine implant and a front reservoir assembled on the front magnet positioned between this one and the tongue implant.

Furthermore, in the fifth aspect of the invention, the device comprises connecting ducts between the matrix reservoir, posterior reservoirs and the front one through which a liquid (fluid) circulates, creating a close circuit.

For the liquid movement from the matrix reservoir to the posterior reservoirs and/or the front reservoir, the device comprises one or more pumps fixed on the support splint which drives the liquid trough the ducts.

For the pumps management in the fifth aspect of the invention, the device comprises a controller and feeding means fixed on the support splint connected with the pumps and the controller.

In a sixth aspect of the invention, the device comprises a front lift assembled below the front magnet which can draw the front magnet closer to the tongue implant, in an enough distance for an attraction force between them.

Besides, the device comprises one or more posterior lifters assembled below the posterior magnets and capable of drawing the posterior magnets near the palatine implant in an enough distance for an attraction force between them.

For the control of the lifters, the device comprises, in the sixth aspect of the invention, a controller in relation with the front lift and the posterior lifters and the feeding means fixed on the support splint, in connection with the controller, the front lift and posterior lifters.

Even the fourth, the fifth and the sixth aspects of the invention, the device can comprise snoring, apnoea and hipoapnoea detection means, in association with the controller.

Alternatively, in another aspect of the invention, the controller comprises a magnetization activation timer. In this way, the device can be programmed for the activation of the front attraction group and/or the palatine posterior attraction group after a while being in the users' mouth. In this way discomfort, excessive salivation and gag reflex are avoided.

The controller can also comprise data communication means associated with an external device as a mobile phone or a computer. The data communication means are configured to send information about the feeding means charge level and the detection means measures taken.

### DESCRIPTION OF THE DRAWINGS

To complete the description in order to trying to help a better comprehension of the invention characteristics, according to a preferred embodiment of the invention, it is accompanied as a part of the description, a drawing ensemble where, and with illustrative and non-limitative character, the following have been represented:
Figure 1.- Shows a lateral section of the oral (or mouth) cavity of a user where the device has been positioned.
Figure 2.- Shows a frontal view of the device positioned in the user's mouth.
Figure 3.- Shows a simplified general overview of the device in an aspect of the invention.
Figure 4.- Shows a superior view of the support and the connection ducts between the reservoirs as the electronic parts of the device in an aspect of the invention.
Figure 5.- Shows a superior view of the support with the ducts that connect the reservoirs and the micro valves, as well as the tongue implant in an aspect of the invention.
Figure 6.- Shows a general overview of the tongue implant.
Figure 7.- Shows a tongue lingual lateral section.
Figure 8.- Shows a general overview of the palatine implant.

### PREFERENT EMBODIMENT OF THE INVENTION

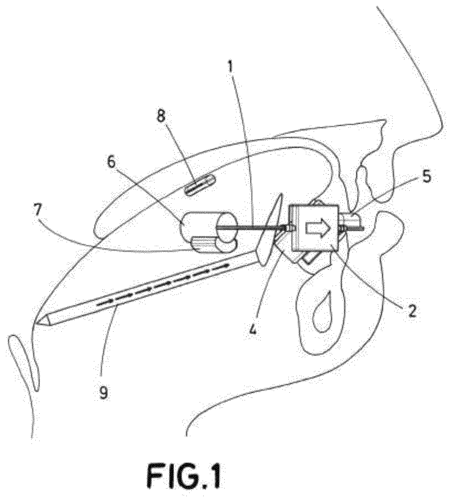

Is described below, with the help of the figures 1 to 8, some embodiments of the device for the snoring and the sleep apnoea and hipoapnoea of the current intervention.

In the figure 1 it is shown a lateral section of the users' mouth in which the device is positioned. As it can be seen, the device comprises a support splint (1) intended to be positioned between both hemiarches. The support splint (1) can be a splint for the superior hemiarch, a splint for the below hemiarch or both. The support splint (1) can also be fixed in the users' mouth if it is a complete or partial scarcity of teething.

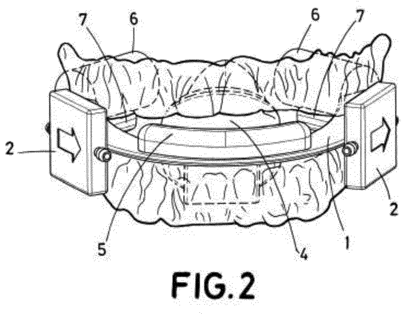

In figure 2 it is shown the device when the support splint (1) comprises a part of the superior and a part of the below hemiarch. In this case and between both, an anterior opening is left.

In any case, the support splint (1) will be completely personalized in relation with the bite characters and the temporomandibular joint movement of the user.

The device also comprises a front attraction group with a front magnet (4) fixed to the support splint (1) and a tongue implant (9) that is intended to be implanted in the users' tongue, having an attraction force between the front magnet (4) and the tongue implant (9).

The front magnet (4) will be positioned in the inner side of the support splint (1) at the same level as the incisor and canine teeth and with an inclination of 45 degrees, occupying the anterior part of the floor of the mouth so the ventral side of the tongue can rest over it. Like that an elongation and lingual overtaking is reached, reducing the volume into a basilingual level.

Even more, the device comprises a posterior attraction group that in turn comprises one or more posterior magnets (7) fixed on the support splint (1) located in the rear position of the upper arch, and a palatine implant (8) intended to be implanted in the palate of a user, existing an attraction and elevation force between the posterior magnets (7) and the palatine implant (8).

In a first aspect of the invention, the device just comprises a front attraction group in case that the user suffers a tongue base collapse. In a second aspect of the invention, the device just comprises the posterior attraction group in case that the user suffers a palatine collapse. In a third aspect of the invention, when the user suffers a tongue and palate collapse the device comprises a front and posterior attraction group. The necessity for implanting one or two implants will be determinated by the collapse localization which brings about the apnoae-hipoapnoea of the user.

The implants (8, 9) should be placed with a minimally invasive surgical approach, with a depth distance from the front magnet (4) and/or rear magnets (7) that allow a perfect trophism between tissues in touch.

In a forth aspect of the invention, the front magnet (4) and/or the posterior magnets (7) are electromagnets which turn on and off when an electric current flows, so once the device is inside the mouth of the user, the moment when the electromagnets or high attraction magnets are activated can be controlled, its attraction force variability depending on the distance between the magnet and the implant.

In the fourth aspect of the invention, the device comprises feeding means (11), fixed on the support splint (1), connected with the front magnet (4) and/or posterior magnets (7) and a controller (12) fixed on the support splint (1) and related with the feeding means (11), the front magnet (4) and posterior magnets (7).

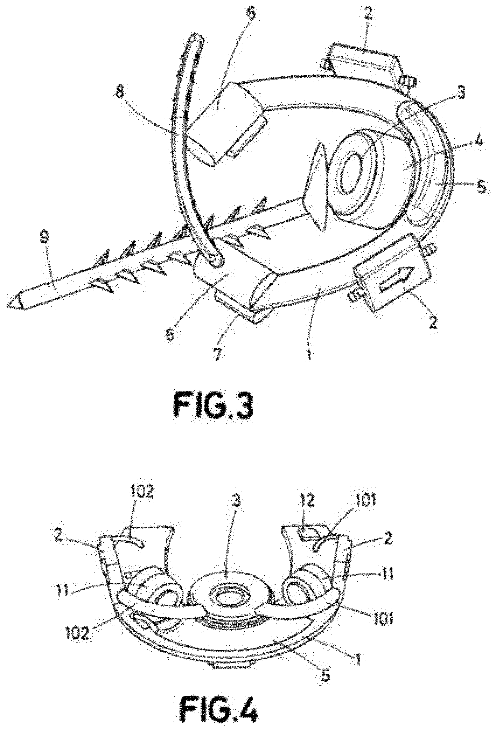

In a fifth aspect of the invention, which is shown with more detail in figure 3, the device comprises a matrix reservoir (5) fixed on the support splint (1), one or more posterior reservoirs (6), assembled on the posterior magnets (7) situated between those and the palatine implant (8), and a front reservoir (3) assembled over the front magnet (4) situated between this one and the tongue implant (9).

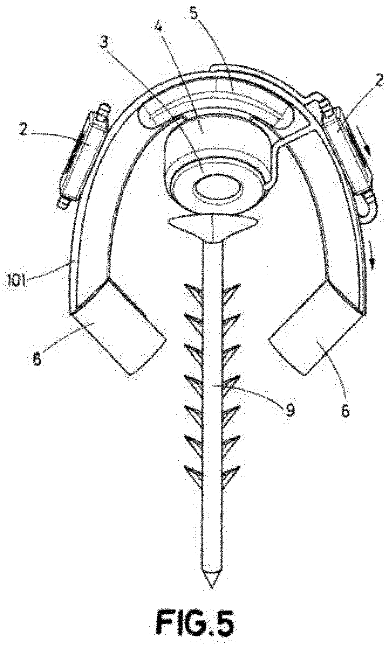

What is more, in the fifth aspect of the invention, and as it is shown in figure 5, the device comprises connection ducts (101) between the matrix reservoir (5) the posterior reservoirs (6) and the front reservoir (3) for which a liquid flows, preferentially physiological saline solution or other biocompatible and non-compressible liquid.

For the movement of the fluid from the matrix reservoir (5) to the posterior reservoirs (6) and/or front reservoir (3) as it is shown in figure 4, the device comprises one or more pumps (2) fixed on the support splint (1) that propel the liquid through the ducts (101).

To manage the pumps (2) the device comprises a controller (12) and feeding means (11) fixed on the support splint (1) connected with the pumps (2) and the controller (12) which are outwardly rechargeable both cabled and wireless. In figure 4 the ducts (101) which connect the different reservoirs (5, 6 and 3) are shown.

In this fifth aspect of the invention, when the posterior reservoirs (6) are liquid full the attraction force between the posterior magnets (7) and the palatine implant (8) does not exist. Besides, there is not an attraction force between the front magnet (3) and the tongue implant (9) when the front reservoir (3) is liquid full. This way, the amount of front attraction and the posterior attraction group only work when all the liquid is in the matrix reservoir (5). This is achieved when the pumps (2) move the liquid from those reservoirs (3, 6) to the matrix reservoir (5).

In a sixth aspect of the invention, the device, additionally comprises a front lift assembled below the front magnet (4) and which is able to draw the front magnet (4) near to the tongue implant (9) in an enough measure for the force attraction between them.

Also, the device comprises one or more posterior lifters assembled below the posterior magnets (7) able to draw the posterior magnets (7) near to the palatine implant (8) in an enough measure for the force attraction between them.

In one embodiment, the lifters could be springs that move the magnets (4, 7) inside a capsule from implants (8, 9) far position to a near implants (8, 9) position.

To control the lifters action, the device comprises a controller (12) in relation with the front lifter and posterior lifters and feeding means (11) fixed on the support splint (1), connected with the controller (12), the front lift and posterior lifters.

Even the fourth as the fifth and sixth aspects of the invention, the device can comprise detection means intended to detect the beginning of apnoea and hipoapnoea, in relation with the controller (12).

Thanks to the detection means, the controller (12) is able to determinate when the front attraction group must be activated and/or the posterior attraction one. In the fourth aspect case, activating the device means making the current flow through the electromagnets. In the fifth aspect of the invention, moving the liquid to the matrix reservoir (5). And in the sixth aspect of the invention, drawing the magnets (4, 7) near the implants (8, 9).

Detection means can be, for example, a microphone for snoring detection or a vibration detector with the same aim. When the snoring of the user is detected the attraction as a whole or/and the repulsion one are activated. The detection means can also be a gyroscope that knows the moment when the user gets in a supine decubitus position and activates the attraction groups. Also a pulse - oximeter that calculates the oxygen in the users' blood.

Alternatively, in other aspect of the invention, the controller (12) comprises an activation timer of the attraction groups. In this way the device can be configured to get activated after a while since the user has it in the mouth. This way uncomfortable, excessive salivation and gag reflex can be avoided.

The controller (12) can also comprise some ways of data communication means in relation with an external device as a computer or a mobile phone. The data communication means send information to those external devices about the feeding means (11) charge level and the measures of the detection means.

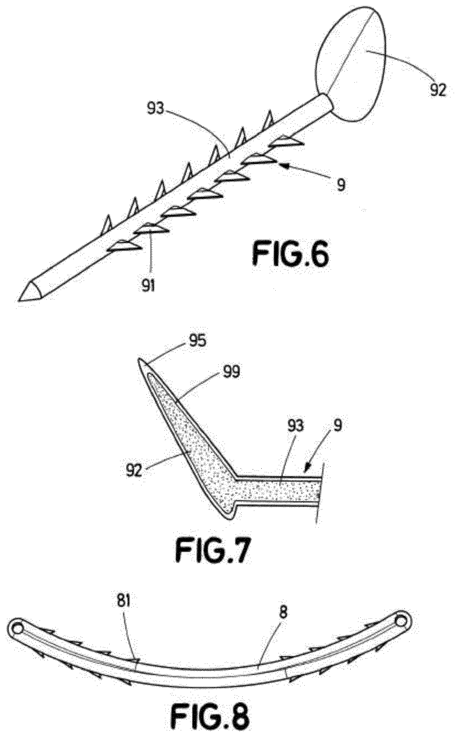

In figure 8 the palatal implant (8) is represented. The palatal implant (8) is intended to be placed on the user's soft palate, from side to side of the user. It comprises a curved central body from which spicules (81) start, which prevent movement within the user's palate.

Specifically, the palatal implant (8) is fixed at each end of the posterior edge of the hard palate, in proximity to the pterygoid hamulus. Fixation is performed using micro-screws made of non - resorbable, biocompatible and bioequivalent metallic material, which are inserted into holes at both ends of the palatal implant (8).

Figure 6 shows the lingual implant (9) in one aspect of the invention. It comprises a first body (92) approximately circular, which is positioned on the ventral face of the user's tongue, and which is the one that suffers a force of attraction towards the front magnet (4).

It also comprises a second elongated body (93) that is located along the tongue and from which spicules (93) start laterally preventing the movement of the second body (93) inside the tongue.

Specifically, the tongue implant (9) is intended to be placed on the ventral side of the tongue, leaving the second body (93) deeply inducted towards the base of the tongue, taking advantage of the anatomical and surgical plane of the lingual septum by the midline. This plane presents the anatomical, advantage of accessing through a fascial a fibrous approach, avoiding vascular and nervous structures of the lateral portions.

The tongue implant (9) as shown in figure 7, as well as the palatal implant (8), comprise and external receptacle (95) and an interior receptacle (94), both of a biocompatible and flexible material, preferably of a biocompatible and bioequivalent silicone elastomer.

Thus, the implants (8, 9) are as ductile and dense as the muscular and mucous tissue in which they are implanted, being completely inactive during the waking face. Furthermore, implants (8, 9) are not bothersome or prevent swallowing and articulation of speech.

A ferromagnetic material (93) is arranged inside the inner receptacle (94), preferably in the form of a powder or gel. When the front magnet (4) or posterior magnets (7) approach the implants (8, 9), the ferromagnetic powder material stiffens, displacing the soft palate and/or tongue anteriorly. Thus, it is possible to simulate through the variable magnetic attraction the tone of the musculature that is abolished during sleep.

In order to achieve a better performance of the implants (8, 9), the ferromagnetic material (93) has zero or almost zero remanence and a high saturation magnetization.

Zero remanence materials are magnetized in the presence of a magnetic field, but once then external magnetic field is removed, the material does not magnetize. In this way, the ferromagnetic material (93) is prevented from the interfering with other elements with a high iron content that may enter the mouth.

On the other hand, the attraction force between magnetic elements increases with its saturation magnetization, the necessary forces can be achieved to maintain muscle tone with smaller implants (8, 9).

In addition to facilitate the extraction of the inner receptacle (94) from the outer receptacle, a biocompatible gel can be placed between them.

## Claims

1. Device for the snoring, and sleep apnoea and hypoapnoea, which comprises:
- a support splint (1),
- at least one of the following:
- a front attraction group, comprising a front magnet (4) attached to the supporting splint (1), and a lingual implant (9), intended to be implanted in the tongue of a user, existing between the front magnet (4) and the lingual implant (9) an attraction force,
- a posterior attraction group, comprising one or more posterior magnets (7) fixed to the support splint (1), and a palatal implant (8), intended to be implanted in the palate of the user, existing an attraction and lifting force between the posterior magnets (7) and the palatal implant (8).

2. The device of claim 1, which comprises the front attraction group and the posterior attraction group.

3. The device of claim 1, wherein the front magnet (4) and / or the posterior magnets (7) are electromagnets, and in which the device additionally comprises:
- feeding means (11) attached to the supporting splint (1), connected to the front magnet (4) and / or to the posterior magnets (7),
- a controller (12) attached to the supporting splint (1) and associated with the feeding means (11), the front magnet (4) and / or the posterior magnets (7).

4. The device of claim 1, which additionally comprises:
- a matrix reservoir (5) attached to the support splint (1),
- at least one of the following:
- one or more posterior reservoirs (6), mounted on the posterior magnets (7), being positioned between these and the palatal implant (8),
- a front reservoir (3), mounted on the front magnet (4), being positioned between it and the lingual implant (9),
- connection ducts (101) between the matrix reservoir (5), the rear reservoirs (6) and the front reservoir (3) through which a fluid runs,
- one or more pumps (2) attached to the support splint (1), which propel the fluid through the ducts (101),
- a controller (12), associated with the pumps (2), and
- feeding means (11) fixed to the support splint (1), connected to the pumps (2) and to the controller (12).

5. The device of claim 1, further comprising:
- at least one of the following:
- a front lift, mounted under the front magnet (4), capable of bringing the front magnet (4) closer to the lingual implant (9),
- one or more posterior lifters, mounted under the posterior magnets (7), capable of drawing the posterior magnets (7) near the palatal implant (8),
- a controller (12), associated to the front lift and / or to the posterior lifters, and
- feeding means (11) fixed to the support splint (1), connected to the controller (12), the front elevator and / or the posterior lifters.

6. The device of claims 3, 4 or 5, additionally comprising detection means configured for detecting entry into apnoea and hypoapnoea, associated with the controller (12).

7. The device of claim 6, wherein the detection means are selected from at least one of microphone, gyroscope, vibration detector and pulse oximeter.

8. The device of claims 3, 4 or 5, wherein the controller (12) additionally comprises an activation timer configured to activate the front attraction group and/or the posterior attraction group.

9. The device of claims 3, 4, or 5, wherein the controller (12) additionally comprises data communication means.

10. The device of claim 1, wherein the lingual implant (9) and / or the palatal implant (8) comprise spicules (81, 92).

11. The device of claim 1, wherein the lingual implant (9) and / or the palatal implant (8) comprise an outer receptacle (95) of a biocompatible material, an inner receptacle (94) and a ferromagnetic material (93) inside the inner receptacle (94).

12. The device of claim 11, wherein the ferromagnetic material (93) is in powder or gel form.

13. The device of claim 12, wherein the ferromagnetic material (93) has zero or almost zero remanence and a high saturation magnetization.

14. The device of claim 11, wherein the outer receptacle (95) and the inner receptacle (94) are made of a flexible material.

15. The device of claim 11, further comprising a biocompatible gel between the outer receptacle (95) and the inner receptacle (94) to facilitate removal of the inner receptacle (94).
